(19) Europäisches Patentamt
European Patent Office
Office européen des brevets

(11) EP 1 023 039 B1

(12) FASCICULE DE BREVET EUROPEEN

(45) Date de publication et mention de la délivrance du brevet:
**14.08.2002 Bulletin 2002/33**

(21) Numéro de dépôt: **98945352.7**

(22) Date de dépôt: **22.09.1998**

(51) Int Cl.$^7$: **A61K 7/48**

(86) Numéro de dépôt international:
**PCT/FR98/02028**

(87) Numéro de publication internationale:
**WO 99/15147 (01.04.1999 Gazette 1999/13)**

# (54) UTILISATION D'ALKYL MONOGLUCOSIDES EN TANT QUE VECTEURS MOLECULAIRES

VERWENDUNG VON ALKYLMONOGLUCOSIDEN ALS MOLEKULARE VEKTOREN

USE OF ALKYLMONOGLUCOSIDES AS MOLECULAR VECTORS

(84) Etats contractants désignés:
**AT BE CH CY DE DK ES FI FR GB GR IE IT LI LU MC NL PT SE**

(30) Priorité: **22.09.1997 FR 9711766**

(43) Date de publication de la demande:
**02.08.2000 Bulletin 2000/31**

(73) Titulaire: **ULICE**
**F-63200 Riom (FR)**

(72) Inventeurs:
- **BOUSQUET, Marie Pierre**
  **F-31200 Toulouse (FR)**
- **WILLEMONT, René-Marc**
  **F-31450 Pompertuzat (FR)**
- **MONSAN, Pierre**
  **F-31700 Mondonville (FR)**
- **BOURES, Emmanuel**
  **F-63000 Clermont-Ferrand (FR)**
- **MESSAGER, Arnaud**
  **F-63200 Riom (FR)**

(74) Mandataire:
**Grosset-Fournier, Chantal Catherine et al**
**Grosset-Fournier & Demachy s.a.r.l.,**
**20, rue de Maubeuge**
**75009 Paris (FR)**

(56) Documents cités:
**WO-A-95/14705**

- **PATENT ABSTRACTS OF JAPAN vol. 017, no. 342 (C-1076), 29 juin 1993 & JP 05 043428 A (LION CORP), 23 février 1993**
- **PATENT ABSTRACTS OF JAPAN vol. 013, no. 011 (C-558), 11 janvier 1989 & JP 63 218631 A (SEKISUI CHEM CO LTD), 12 septembre 1988**
- **T. HALMOS: "Synthesis of glucose-chlorambucil derivatives and their recognition by the human GLUT1 glucose transporter" STN INTERNATIONAL, KARLSRUHE, FILE CHEMICAL ABSTRACTS, AN=126:176810, XP002069798 & EUR. J. PHARMACOL. (1996), 318(2/3), 477-484,**
- **H. NISHIO: "Hydantoin-containing glycosides, UV absorbents, and skin preparations containing them" STN INTERNATIONAL, KARLSRUHE, FILE CHEMICAL ABSTRACTS, AN=128:26765, XP002069799 & JP 09 278788 A (KANEBO, LTD.)**
- **T. IKEMOTO: "Preparation of cinnamic acid alkyl glycoside esters, ultraviolet ray absorbent and skin topical application" STN INTERNATIONAL, KARLSRUHE, FILE CHEMICAL ABSTRACTS, AN=127:331692, XP002069800 & JP 09 268194 A (KANEBO, LTD.)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 023 039 B1

## Description

La présente invention concerne l'utilisation d'alkylmonoglucosides et plus particulièrement du n-butyl-α-D-monoglucopyranose, ci-après dénommé α-butylglucoside comme vecteur moléculaire.
Nous avons constaté qu'il existait un besoin de modifier de nombreux actifs cosmétiques ou pharmaceutiques et/ou ingrédients alimentaires afin d'améliorer :

- leur biodisponibilité
- leur toxicité
- leur liposolubilité
- leur hydrosolubilité

La présente invention se propose de répondre à ces besoins. Elle concerne l'utilisation, dans les domaines pharmaceutique, dermatologique, cosmétique ou alimentaire, d'alkylmonoglucosides ayant la formule générale :

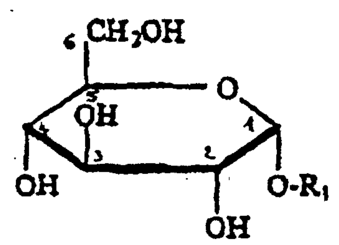

dans laquelle $R_1$ est un radical alkyle, linéaire ou ramifié en $C_2$ à $C_{18}$, en tant que vecteur moléculaire transcutané ou transmuqueuse d'un composé actif étant greffé par une liasion covalente à la position 6 dudit alkylmonoglucoside.
Par "vecteur moléculaire", on veut dire un composé, qui après réaction chimique avec un composé actif donne un composé actif vectorisé qui pénètre plus facilement la peau ou une muqueuse que le composé actif de départ.
Dans ces actifs vectorisés, la molécule active est liée par covalence à la position 6 du vecteur. L'invention est issue du fait que nous avons réduit la toxicité d'agents exfoliants utilisés en cosmétique et/ou dermatologie par greffage sur l'α-butylglucoside.
Le document D1 (Patent Abstracts of Japan, Vol. 013, no. 011 (C-558), 11.01.89, JP 63 218631 A) décrit l'association d'alkylglucosides, notamment de D-(thio)-glucosides, avec un composé actif pour préparer une composition percutanée. Cette association concerne un simple mélange entre le composé actif et l'alkylglucoside, et non un composé actif vectorisé résultant du greffage de l'alkylglucoside sur le composé actif.
Le document D2 (WO 95/14705) décrit l'utilisation d'α-D-alkylglucopyranosides et d'esters de ceux-ci pour la préparation de prodrogues capables de traverser la barrière hémato-encéphalique. Les composés α-D-alkylglucopyranosides décrits dans ce document portent au niveau de leur carbone 6, deux atomes d'hydrogène et un groupe $OR_2$ dans lequel $R_2$ est un groupe -CO-R où R est un radical hydrocarboné, linéaire ou ramifié, saturé ou éthyléniquement insaturé, en $C_5$ à $C_{17}$, ou un groupe $-CO-(CH_2)_n-COOH$ où n = 4 à 14, et sont plus particulièrement utilisés en tant que vecteurs de médicaments vers le système nerveux central, à travers la barrière hémato-encéphalique.
Les compositions cosmétiques et/ou dermatologiques ont pour vocation, entre autres, d'agir sur la fonction de protection de la peau ce qui implique d'influer directement sur l'état de la couche cornée. Il est connu que si cette couche cornée contient trop de cellules mortes, elle ne protège plus. Il faut alors l'en débarrasser pour permettre à une nouvelle couche de cellules d'assurer une barrière efficace contre les agressions externes, et aux actifs cosmétiques et dermatologiques de la pénétrer. Tel est le rôle connu et dévolu aux Alpha Hydroxy Acides ou AHA.
Les AHA sont des acides organiques avec une fonction alcool sur le carbone voisin de celui (en position alpha) portant la fonction acide carboxylique. Nous regroupons dans cette famille de composés plus particulièrement l'acide glycolique, l'acide lactique, l'acide malique, l'acide tartrique, l'acide citrique, l'acide gluconique ainsi que certains analogues d'AHA comme l'acide salicylique et la serine.
Ces AHA ont une efficacité reconnue et mesurable mais ils présentent aussi quelques inconvénients. Les AHA sont souvent irritants et ont une mauvaise biodisponibilité : ils pénètrent parfois trop rapidement dans les couches profondes de la peau. Dans Parfums Cosmétiques Arômes 122, 66-72 (Avril, Mai 1995), il est décrit les améliorations proposées par les fournisseurs de matières premières cosmétiques pour rendre ces composés moins irritants et/ou freiner leur pénétration dans la peau.
Une tentative d'amélioration proposée consiste à encapsuler ces AHA dans des capsules afin de ralentir la diffusion des AHA. Malheureusement, il est difficile de connaître avec exactitude le pourcentage d'actifs encapsulés

et encore plus difficile d'évaluer le pourcentage d'actifs encapsulés libérés dans la peau.

Une autre tentative consiste à lipophiliser ces AHA en greffant par estérification un composé lipophile (alcool gras, chaînes alkyles). Cependant, l'action de ces composés se voit réduite en raison de leur caractère lipophile. En effet, ils ne diffusent que très difficilement dans le stratum corneum dans lequel ils sont arrêtés par la présence de compartiments aqueux dans les espaces intercornéocytaires.

Nous avons étendu cette réflexion aux acides gras saturés et insaturés. En effet, la couche cornée est constituée par une masse compacte de 20 couches de cellules inactives, enchâssées dans un système de double-couches lamellaires de lipides. Cette structure du stratum corneum ainsi que la nature lipophile de la barrière lipidique protège la peau contre le dessèchement provoqué par la perte insensible d'eau transépidermale. Les compositions cosmétiques et/ou dermatologiques ont pour vocation, entre autres, d'agir sur la fonction de protection de la peau et d'en améliorer l'aspect. Si les lipides intercellulaires de la couche cornée sont altérés, la peau ne protège plus.

Les acides gras insaturés, tels que l'acide linoléique et l'acide linoléique, sont un facteur important pour la construction et la réparation de la barrière lipidique. Ils fonctionnent comme des molécules précurseurs pour la synthèse d'une substance-signal qui contrôle la prolifération et l'activité des cellules.

De plus, les acides gras insaturés participent aussi directement à la régulation de la perméabilité cutanée. Ce sont des substances lipophiles peu occlusives capables de réaliser un film plus ou moins continu à la surface cutanée mais surtout susceptibles de s'incorporer au ciment intercellulaire jouant ainsi un rôle actif dans la régulation de l'hydratation. Leur activité biologique est réglée par la position de la double liaison la plus proche du groupe terminal méthylique.

Les cas observés cliniquement d'altération cutanée comme l'acné montrent qu'un apport suffisant en acides gras insaturés dans la peau est nécessaire pour maintenir le fonctionnement de la barrière lipidique.

Les acides gras insaturés ont donc un rôle primordial dans la physiologie de la peau. Leur administration par voie topique pose cependant des problèmes, problèmes que nous nous proposons de résoudre en les vectorisant.

L'action de ces composés se voit réduite car ils ne diffusent que très difficilement dans le stratum corneum : ils sont arrêtés par la présence de compartiments aqueux contenus dans les espaces intercornéocytaires. Le greffage de ces acides sur l'$\alpha$-butylglucoside augmente le caractère hydrophile et optimise donc la pénétration de ces actifs dans l'épiderme.

De plus, la pénétration de ces compositions dans l'épiderme pose problème en raison de leur caractère lipophile. Leur introduction dans des émulsions stabilisées par une monocouche de tensioactifs n'améliore pratiquement pas cet état de fait étant donné que ces émulsions se cassent dès leur application sur la peau. Il reste alors à la surface de la peau une phase huileuse contenant les acides gras insaturés. Grâce à l'invention l'augmentation du caractère hydrophile par les fonctions hydroxyles libres de la partie glucose améliore la pénétration et permet une utilisation optimisée des acides gras insaturés dans des émulsions eau dans huile ou huile dans eau.

Par extension, ce principe peut s'appliquer à de nombreux composés actifs lipophiles ayant une action physiologique sur la peau. A titre d'exemple, nous pouvons citer les dérivés estérifiables des vitamines lipophiles A, D, E ou F, des huiles essentielles, des filtres solaires, des anti-inflammatoires ainsi que des agents bio-stimulants des synthèses de lipides et/ou protéines. Le document FR-A-94 12005 expose différentes solutions consistant à préparer des émulsions huile dans eau spécifiques.

Certains actifs cosmétiques, dermatologiques, pharmaceutiques et/ou ingrédients alimentaires sont instables car sensibles à des facteurs extérieurs comme la lumière ou la chaleur.

Aussi, différents moyens ont été utilisés pour stabiliser ces composés. Un de ces moyens réside par exemple à bloquer le site sensible par estérification avec des dérivés phosphatés, sulfatés, alkylés et à employer ces dérivés au lieu du composé non modifié. Or ces dérivés ont une activité moins bonne et peuvent parfois être plus toxiques que l'actif libre par la présence de résidus phosphatés, sulfatés ou alkylés.

Un autre moyen consiste à bloquer le site par un dérivé glycosidique. Il est ainsi obtenu un précurseur d'actifs qui après application sur la peau est coupé par les enzymes cutanées ou après administration par voie orale est hydrolysé. L'actif libre est alors libéré. Ainsi, le brevet EP-A-627441 décrit la préparation et l'utilisation de glucosylate d'acide ascorbique, par voie topique, coupé par les enzymes de la peau qui libèrent donc l'acide ascorbique. Mais l'utilisation de tels dérivés entraîne aussi la libération de glucose à la surface de la peau qui favorise le développement de la flore pathogène cutanée.

La Demanderesse a maintenant trouvé de manière inattendue que certains alkylmonoglucosides et plus particulièrement l'$\alpha$-butylglucoside, permettaient toutes ces améliorations tout en évitant les problèmes décrits dans l'art antérieur.

La présente invention a donc pour principal objet l'utilisation de dérivés d'alkylmonoglucosides et plus particulièrement d'$\alpha$-butylglucoside dans une composition alimentaire, cosmétique, dermatologique ou pharmaceutique.

Les alkylglucosides, plus particulièrement l'$\alpha$-butylglucoside, ainsi que certains esters d'alkylglucosides, peuvent être obtenus par voie enzymatique tel que décrit dans la demande de brevet PCT/FR92/00782 appartenant à la Demanderesse.

Les produits issus de ce procédé sont anomériquement purs (α) et sont des monoglucosides. En raison de la quasi absence d'anomère β, les composés ainsi obtenus, ont des propriétés physico-chimiques spécifiques, telles que le point de fusion et la solubilité. Le caractère amphiphile rend l'α-butylglucoside miscible en toutes proportions avec les acides gras saturés et insaturés en milieu fondu et soluble dans les milieux aqueux et solvants organiques polaires de synthèse.

Tableau n° 1 :

| solubilité de l'α-butylglucoside dans l'eau et dans différents solvants. | |
|---|---|
| Solvant | Solubilité à 20°C % (p/p) |
| Eau | 60 |
| Ethanol (95/96%) | 70 |
| Glycérol | 70 |
| Acétonitrile | 50 |
| Dichlorométhane | 70 |
| Dioxane | 70 |
| Polyéthylène glycol 200 | 70 |
| Propylène glycol | 70 |

Nous pouvons modifier les actifs et/ou ingrédients par l'α-butylglucoside pour les rendre lipophiles ou hydrophiles. Il devient alors possible de moduler leur comportement physico-chimique ainsi que leur pénétration, ce qui permet de très fortement diminuer tout risque d'irritation.

Les actifs possédant au moins une fonction acide ou ester peuvent être greffés avec l'aide d'un catalyseur chimique ou enzymatique, notamment les trialglycérol hydrolases (E.C. n° 3. 1.1.3) qui peuvent agir comme carboxyestérase. Nous avons utilisé le Novozym® ou Lipozyme® car il s'agit d'enzymes commerciales, facilement accessibles. Les actifs auxquels s'applique l'invention concerne ceux comportant, par exemple :

- Au moins une fonction acide et plus particulièrement les amino et les α-hydroxy acides tels que l'acide glycolique, l'acide lactique, l'acide malique, l'acide tartrique, l'acide citrique; l'acide gluconique, l'acide salicylique et la serine.
- Une fonction acide et notamment l'acide butyrique, les acides gras saturés et insaturés, et plus particulièrement l'acide oléique, l'acide érucique, l'acide ricinoléïque, l'acide linoléique , et l'acide alpha et gamma linolénique.
- Au moins une fonction ester comme le lactate de méthyle, le lactate d'éthyle, le lactate de butyle ou tout autre dérivé estérifié des acides mentionnés ci-dessus. Il peut s'agir d'esters de cétone et notamment d'ester de dihydroxyacétone. L'ester utilisé selon l'invention comporte une ou plusieurs fonctions ester à chaîne linéaire ou ramifiées, saturée ou insaturée, ayant de 2 à 25 atomes de carbone, comportant éventuellement un ou plusieurs substituants.
- Au moins une fonction carboxylique estérifiable, et notamment, des dérivés de vitamines telles que le rétinol (vitamine A) et ses dérivés (notamment l'acide rétinoïque), d'acide ascorbique (vitamine C) et ses dérivés, des tocophérols, dont la vitamine E et les vitamines D, ainsi que les acides aminés, les peptides et leurs dérivés.. Il peut aussi s'agir de dérivés de polyphénols et plus particulièrement des dérivés polyhydroxylés de flavane et spécialement de flavan-3-ol.

Par actifs vectorisés, nous entendons le couplage par une liaison chimique covalente de l'actif ou ingrédient à la position 6 d'un alkylmonoglucoside et plus particulièrement à l'α-butylglucoside, de formule

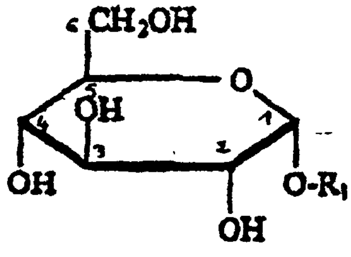

dans laquelle $R_1$ est un radical alkyle, linéaire ou ramifié en $C_2$ à $C_{18}$, de préférence un radical butyle.

Les actifs vectorisés peuvent être utilisés en une quantité allant de 0,1 à 50% en poids, et de préférence de 0,5 à 50% en poids quand il s'agit d'ester de cétones vectorisés et notamment d'ester de dihydroxyacétone vectorisé et de 0,1 à 10% en poids quand il s'agit des autres actifs vectorisés.

A titre d'exemple, et dans que cela soit considéré comme limitatif, on décrit ci-dessous la préparation de deux types de dérivés.

Exemple 1 : Préparation d'ester lactique d'α-butylglucoside.

Dans un ballon adaptable à un évaporateur rotatif, on place 120g d'α-butylglucoside dans 1 litre de L-lactate de butyle à 60°C sous vide. On ajoute 100g de Novozym®, de la lipase de Candida antarctica immobilisée sur support solide (Novo Industri, Danemark). La solution est remise sous vide (15 mbars) à 60°C pendant au moins 48 heures.

En fin de réaction, le milieu réactionnel est centrifugé et filtré pour éliminer complètement le Novozym®. Le filtrat est recueilli pour purification. Le lactate d'α-butylglucoside est purifié par deux extractions successives à l'hexane. Le lactate de butyle est extrait dans la phase organique. Le lactate d'αbutylglucoside est récupéré dans la phase aqueuse qui est décolorée au charbon actif. 2,5% (p/v) de charbon actif est ajouté à une solution molaire de lactate d'αbutyl glucoside. Le tout est ensuite laissé à incuber sous agitation, pendant 3 heures à 60°C, puis filtré pour éliminer le charbon actif.

Exemple 2 : Préparation d'ester lactique d'α-butylglucoside.

Dans un ballon adaptable à un évaporateur- rotatif, on place de l'α-butylgiucoside (100mM) et du L-lactate de méthyle (500 mM) dans 20 ml de méthyl-2 butanol à 60°C sous vide. On ajoute 100g/l de Novozym®, de la lipase de Candida antarctica immobilisée sur support solide (Novo Industri, Danemark). La solution est remise à 60°C sous vide (200 mbars).

Le filtrat est recueilli pour purification. Le lactate d'α-butylglucoside est purifié par deux étapes successives. Le Novozym® est éliminé par filtration. Le lactate de méthyle et le méthyl-2-butanol-2 sont éliminés par évaporation sous vide (80°C - 10 mbars).

Exemple 3 : Préparation de 6-O-oléyl-α-butylglucoside par estérification enzymatique.

Un milieu a été constitué avec 1,2g d'acide oléique et 1g d'α-D-butylglucoside. Le mélange est porté à 65°C, température de fusion de l'α-butylglucoside. Après homogénéisation, on ajoute 1g de Lipozyme®, de la lipase de Mucor miehei immobilisée sur support solide (Novo Industri, Danemark).

Après 3 jours d'incubation, le milieu réactionnel est dilué avec 20 ml d'éther diéthylique, puis filtré de façon à éliminer le Lipozyme®. Après filtration, on ajoute 50 ml de soude (0,02N), de façon à solubiliser dans la phase aqueuse l'acide oléique résiduel sous la forme d'un sel de sodium, ainsi que l'α-butylglucoside résiduel.

La phase organique contenant l'ester de l'acide oléique est évaporée sous vide de façon à éliminer toute trace de solvant résiduel.

En variante, du Novozym®, à une concentration de 5% en poids par rapport aux substrats, a été substitué au Lipozyme®. Egalement un oléate d'alkyle inférieur (méthyle ou éthyle, par exemple) peut être substitué à l'acide oléique.

Des mesures par HPLC ont montré que plus de 95% de l'acide peut être converti quels que soient l'enzyme et le composé acylé choisis. Cependant l'utilisation de Novozym® comme biocatalyseur mène aux plus hautes conversion avec plus de 98% des deux substrats qui ont été convertis.

Le contrôle de la réaction par Chromatographie Couche Mince (CCM) a montré une formation d'un produit unique quand le Novozym® a été utilisé comme biocatalyseur.

La structure des produits a été déterminée par Spectroscopie de Masse (MS) Résonance Nucléaire Magnétique (NMR).

Après l'acylation de α-butylglucoside avec de l'acide oléique avec comme catalyseur Novozym®, le produit a été purifié.

Les données de MS montrent un ion moléculaire à m/z = 523,3 $[M + Na]^+$, M correspond exactement à la masse moléculaire du monooléate de α-butylglucoside. Les données de NMR ont démontré que l'α-butylglucoside a été exclusivement greffé sur la position 6 de la molécule.

Le déplacement chimique du proton à 5,42 ppm confirme la présence d'une insaturation carbone-carbone venant de la partie oléique de la molécule 6-O-oléyl α-butylglucoside. Les indices d'iodé du milieu de réaction au début (mélange de l'acide oléique et α-butylglucoside) et à la fin (6-O-oléyl α-butuylglucoside) de l'estérification enzymatique sont les mêmes à moins de 3% d'erreur (43,7 et 42,7 respectivement). Cela démontre qu'aucune oxydation de la double liaison ne s'est produite pendant le processus de l'estérification enzymatique.

Exemple 4 : Synthèse d'un mélange d'esters de l'α-butylglucoside.

On a procédé à l'acylation d'α-butylglucoside par un mélange commercial d'acide linoléique (60,5% molaires), d'acide oléique (32,7% molaires) et d'acide linolénique (6,8% molaires) en l'absence de solvant, à 60°C sous une pression réduite de 20 mbars, avec un rapport moléculaire initial acides/α-butylglucoside de 1, et en présence de Novozym® à une concentration initiale de 5% en poids par rapport au poids total des réactifs acides et α-butylglucoside.
L'eau générée par la réaction a été évaporée sous la pression réduite établie, afin de déplacer l'équilibre thermodynamique en faveur de la synthèse d'esters.
On a récupéré les esters produits en ajoutant un peu d'hexane puis en en éliminant l'enzyme par filtration.
La conversion des acides de départ en esters de α-butylglucoside a été presque totale (> 95%).
A titre d'exemple, et sans que cela soit considéré comme limitatif, on décrit ci-dessous l'amélioration de l'irritation cutanée et oculaire engendrée par le greffage d'une préparation sur l'α-butylglucoside.

Exemple 5 : Comparaison de la cytotoxicité de l'acide lactique et du lactate d'α-butylglucoside.

Nous avons quantifié et comparé, in vitro, le cytotoxicité de l'acide lactique et du lactate d'α-butylglucoside sur un modèle de peau. Ce modèle de peau est constitué d'une matrice de collagène sur laquelle repose un épiderme reconstitué à partir de kératinocytes et présentant une architecture similaire à celle de l'épiderme humain. La transformation du sel de tétrazolium (MTT) en cristaux bleus de formozan est proportionnelle à l'activité de la succinate déshydrogénase, (une enzyme mitochondriale). En conséquence, plus l'épiderme contiendra de cellules vivantes, plus la transformation par la succinate déshydrogénase du MTT en cristaux bleu de formozan sera importante. La quantité de formozan est mesurée au spectrophotomètre. La viabilité cellulaire, réalisée pour déterminer la toxicité du produit, est calculée selon la formule

$$\% \text{ viabilité} = \text{DO produit}/\text{DO témoin cellulaire} \times 100$$

Le témoin cellulaire est réalisé sans produit. Un tensio-actif de référence SDS (Sodium Dodecyl Sulfate) à 1,5 mg/ml est également testé comme témoin cytotoxique.

- Si la viabilité cellulaire est comprise entre 70 et 50%, les produits sont considérés comme légèrement cytotoxiques.

Si elle est inférieure à 50%, les produits sont considérés comme cytotoxiques.
Le tableau 2 présente les résultats de l'évaluation.

Tableau n° 2 :

| dosage MTT sur modèle de peau densités optiques obtenues à 540 nm et pourcentage de viabilité des cellules épidermiques par rapport au témoin | | | | |
|---|---|---|---|---|
| Essai | Témoin | SDS à 1,5 mg/ml | Acide lactique à 5% | Lactate d'α-butylglucoside à 5% |
| DO essai 1 | 0,324 | 0,046 | 0,044 | 0,242 |
| DO essai 2 | 0,363 | 0,056 | 0,066 | 0,265 |
| Moyenne | 0,343 | 0,051 | 0,055 | 0,255 |
| % viabilité par rapport au témoin | 100% | 14,8% | 16% | 74,5% |

Le lactate d'α-butylglucoside, acide lactique vectorisé, est moins irritant que l'acide lactique. Le lactate d'α-butylglucoside à 5% n'est pas cytotoxique.

Exemple 6 : Comparaison de la toxicité oculaire de l'acide lactique et du lactate d'α-butylglucoside (acide lactique vectorisé).

Le test BCOP (opacité et perméabilité cornéenne bovine) permet d'évaluer l'indice d'irritation oculaire in vitro sur des cornées provenant de boeufs d'abattoir. Elle mesure deux paramètres de l'irritation oculaire : l'opacité et la perméabilité cellulaire.

L'opacité est définie comme la différence de transmission lumineuse entre une cornée traitée et une cornée témoin. La perméabilité est égale à la densité optique (DO) mesurée au spectrophotomètre à 490 nm.

Le score d'irritation in vitro est établi en utilisant la formule suivante :

Score in vitro = valeur d'opacité + 1,5 fois la valeur de DO.

Les valeurs objectives sont combinées et les scores in vitro d'irritation oculaire sont comparés à une échelle d'irritation établie précédemment. En général, le potentiel irritant est classé en trois catégories faible (0 à 25), modéré (25,1 à 55) et sévère (55,1 et +).

L'indice d'irritation oculaire in vitro du lactate d'α-butylglucoside à 36% est nul alors que ceux de l'acide lactique à 5% et 10% sont, respectivement de 1,93 et de 41,11.

Notre préparation de lactate d'α-butylglucoside à 36% appartient donc à la catégorie des produits non irritants, tandis que l'acide lactique à 10% appartient à la catégorie des produits moyennement irritants.

## Revendications

1. Utilisation dans les domaines pharmaceutique, dermatologique, cosmétique et alimentaire, d'un alkylmonoglucoside ayant la formule générale :

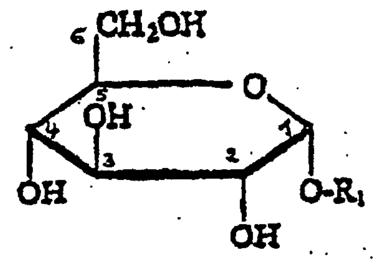

dans laquelle $R_1$ est un radical alkyle, linéaire ou ramifié en $C_2$ à $C_{18}$,

en tant que vecteur moléculaire transcutané ou transmuqueuse d'un composé actif, ledit composé actif étant greffé par une liaison covalente à la position 6 dudit alkylmonoglucoside.

2. Utilisation selon la revendication 1, **caractérisée par le fait que** le groupe R1 de l'alkylmonoglucoside est un radical butyle.

3. Utilisation selon la revendication 1 ou la revendication 2 d'un alkylmonoglucoside ayant la formule générale

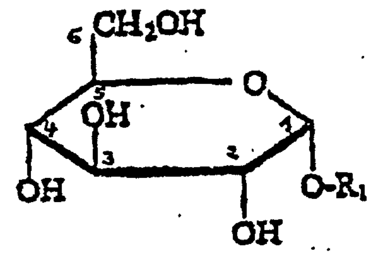

dans laquelle $R_1$ est un radical alkyle, linéaire ou ramifié en $C_2$ à $C_{18}$,

en tant que vecteur moléculaire transcutané ou transmuqueuse d'un composé actif, ledit composé actif étant greffé par une liaison covalente à la position 6 le dudit alkylmonoglucoside, afin d'améliorer la biodisponibilité, la toxicité, la liposolubilité et/ou l'hydrosolubilité de composés actifs pharmaceutiques, cosmétiques et alimentaires.

4. Utilisation selon la revendication 3, **caractérisée par le fait que** le groupe $R_1$ est un radical butyle.

## Claims

1. Use in pharmaceutical, dermatological, cosmetic and food domains, of an alkylmonoglucoside with the general formula:

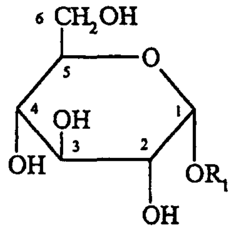

in which $R_1$ is a $C_{2-18}$, linear or branched alkyl radical,
as a transcutaneous or transmucous membrane molecular vector of an active compound, said active compound being grafted by a covalent link at the position 6 of said alkylmonoglucoside.

2. Use according to claim 1, **characterised by** the fact that the $R_1$ group of the alkylmonoglucoside is a butyl radical.

3. Use according to claim 1 or claim 2, of an alkylmonoglucoside With the general formula:

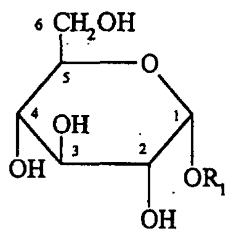

in which $R_1$ is a $C_{2-18}$, linear or branched alkyl radical,
as a transcutaneous or transmucous membrane molecular vector of an active compound, said active compound being grafted by a covalent link at the position 6 of said alkylmonoglucoside, in order to improve the bioavailability, the toxicity, the liposolubility and/or the hydrosolubility of pharmaceutical, cosmetic and food active compounds.

4. Use according to claim 3, **characterised by** the fact that the group $R_1$ is a butyl radical.

## Patentansprüche

1. Verwendung auf dem pharmazeutischen, dermatologischen, kosmetischen und Lebensmittel- Gebiet eines Alkylmonoglucosids, das folgende allgemeine Formel aufweist:

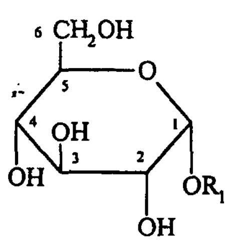

in welcher $R_1$ ein linearer oder verzweigter Alkylrest mit 2 bis 18 Kohlenstoffatomen ist,

als über die Haut oder Schleimhaut verabreichbarer molekularer Vektor einer aktiven Verbindung, wobei die aktive Verbindung über eine kovalente Bindung an der Position 6 des Alkylmonoglucosids gebunden ist.

**2.** Verwendung gemäß Anspruch 1, **dadurch gekennzeichnet, dass** die Gruppe R1 des Alkylmonoglukosids ein Butylrest ist.

**3.** Verwendung gemäß Anspruch 1 oder 2 eines Alkylmonoglucosids, das folgende allgemeine Formel aufweist:

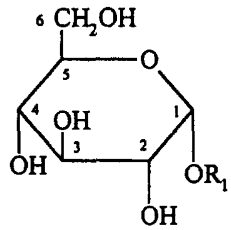

in welcher $R_1$ ein linearer oder verzweigter Alkylrest mit 2 bis 18 Kohlenstoffatomen ist,

als über die Haut oder Schleimhaut verabreichbarer molekularer Vektor einer aktiven Verbindung, wobei die aktive Verbindung über eine kovalente Bindung an der Position 6 des Alkylmonoglucosids gebunden ist, um die Bioverfügbarkeit, die Toxizität, die Fettlöslichkeit - und/oder die Wasserlöslichkeit der aktiven pharmazeutischen, kosmetischen und Lebensmittel- Verbindungen zu verbessern.

**4.** Verwendung gemäß Anspruch 3, **dadurch gekennzeichnet, dass** die Gruppe $R_1$ des Alkylmonoglukosids ein Butylrest ist.